(19) Europäisches Patentamt
European Patent Office
Office européen des brevets

(11) **EP 0 729 742 B1**

(12) **EUROPEAN PATENT SPECIFICATION**

(45) Date of publication and mention of the grant of the patent:
**06.11.2002 Bulletin 2002/45**

(51) Int Cl.[7]: **A61K 7/06**

(21) Application number: **96102981.6**

(22) Date of filing: **28.02.1996**

# (54) Hair cosmetic composition containing an amino-modified silicon polymer

Haarkosmetische Zusammensetzung, die ein aminomodifiziertes Siliconpolymer enthält

Composition cosmétique pour cheveux contenant un polymère à base de silicium modifié par des groupes amino

(84) Designated Contracting States:
**DE FR GB**

(30) Priority: **01.03.1995 JP 4179095**

(43) Date of publication of application:
**04.09.1996 Bulletin 1996/36**

(73) Proprietor: **Kao Corporation**
**Chuo-Ku Tokyo 103 (JP)**

(72) Inventors:
- **Yahagi, Kazuyuki**
  **Tokyo (JP)**
- **Tashiro, Kazuhiro**
  **Yokohama-shi, Kanagawa (JP)**
- **Kuwata, Satoshi, c/o Shin-Etsu Chem. Co., Ltd.**
  **Annaka-shi, Gunma (JP)**
- **Nakazato, Morizo, c/o Shin-Etsu Chem. Co., Ltd.**
  **Annaka-shi, Gunma (JP)**

(74) Representative:
**Kindler, Matthias, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 531 763**
**EP-A- 0 532 256**
**GB-A- 2 058 103**
**GB-A- 2 165 550**
**GB-A- 2 173 515**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

Field of the Invention:

The present invention relates to a hair cosmetic composition which can impart smoothness, softness and antistatic effect to hair, permits good finish that the hair becomes soft, moist and innocent of oil or not sticky to the touch, makes the hair hard to become disheveled hair during sleep and hence has excellent retention of hairstyle set, possesses high remaining ability on the hair, so that it can fully exhibit its effects even on hair damaged by permanent waving, blow-dry and/or the like, and is also excellent in stability.

Description of the Background Art:

In order to impart softness, smoothness, antistatic effect and the like to hair, there have heretofore been used hair cosmetic compositions in which a cationic non-silicone polymer and/or a cationic surfactant as a conditioning component is incorporated in combination with an oily base such as a higher alcohol, glyceride, liquid paraffin or ester. However, the combined use of these oily bases can enhance the softness and smoothness compared with the single use of the cationic non-silicone conditioning component, but involves a drawback that it is unavoidable to give a feeling that the oily base remains, namely, have a oily and sticky feel.

On the other hand, silicones typified by dimethyl polysiloxane have been known to be smooth and excellent in lubricity compared with the oily bases such as higher alcohols, glycerides, liquid paraffin and esters and give a feeling innocent of oil and gloss to hair, and are used in many hair cosmetic compositions. However, such silicones have failed to impart sufficient performance as to softness and retention of hairstyle set. In particular, they have involved a drawback that their adsorbing and remaining ability on hair is lowered for hair damaged by permanent waving, hair coloring and/or the like, and so their effects cannot be fully exhibited.

As described above, the silicones heretofore used have no adsorbing group to hair. As a result, in order to fully exhibit their performance, they must be incorporated in a great amount into hair cosmetic compositions. Therefore, the resulting compositions have involved a drawback that they are sticky to the touch at the roots and the like of the hair not very damaged when applied. In addition, the incorporation of the silicones in a great amount has also offered a problem that products in which the silicones have been incorporated are deteriorated in stability.

Further, the conventional hair cosmetic compositions have involved a problem that their effects do not last long due to shampooing or the like. In recent years, it has thus been attempted to use a modified silicone high in adsorptivity on hair so as to develop a hair cosmetic composition intended for the improvement of lastingness (for example, Japanese Patent Application Laid-Open Nos. 66506/1980, 45406/1981, 210005/1982, 74602/1983, 36407/1985, 56916/1985, 6/1986, 78710/1986, 275515/1988, 307810/1988, 307811/1988, 172313/1989, 190619/1989, 203314/1989, 273609/1990, 273612/1990, 502286/1990, 206022/1991, 36225/1992, 85918/1993, etc.).

However, all these hair cosmetic compositions have involved a problem that good effects can be given to healthy hair, while a moisturized feeling and hence a pleasant feeling cannot be given to permed hair and damaged parts at the tip of the hair, or to the contrary, good effects can be given to the damaged hair, while a sticky feel is given to the healthy hair.

As described above, there has heretofore been no hair cosmetic composition which can impart a function inherent in a silicone to hair portions not very damaged and at the same time, fully exhibit its effects even on hair portions damaged considerably, and has excellent remaining ability even on hair in any conditions and superb retention of hairstyle set in that it makes hair hard to become disheveled hair during sleep.

SUMMARY OF THE INVENTION

Accordingly, it is an object of the present invention to provide a hair cosmetic composition which can impart smoothness, softness and antistatic effect to hair, permits good finish, has excellent retention of hairstyle set and high remaining ability on the hair, and also possesses excellent stability.

In view of the foregoing circumstances, the present inventors have carried out an extensive investigation. As a result, it has been found that when a specific amino-modified terpolymer is used in combination with a cationic non-silicone conditioner, a hair cosmetic composition, which can impart smoothness, softness and antistatic effect to hair, permits good finish that the hair becomes soft, moist and innocent of oil or not sticky to the touch, makes the hair hard to become disheveled hair during sleep and hence has excellent retention of hairstyle set, possesses high remaining ability on the hair, so that it can fully exhibit its effects even on hair damaged by permanent waving, blow-dry and/or the like, and is excellent in stability, can be obtained, thus leading to completion of the present invention.

According to the present invention, there is thus provided a hair cosmetic composition comprising the following components (A) and (B):

(A) an amino-modified terpolymer having structural units represented by the formulae (1), (2) and (3):

$$[(R^1)_2SiO] \quad (1);$$

$$[R^2R^3SiO] \quad (2);$$

and

$$[R^4SiO_{3/2}] \quad (3)$$

wherein $R^1$, $R^2$ and $R^4$ are independently a monovalent hydrocarbon group having 1-20 carbon atoms, and $R^3$ is a group, $-R^5-(NR^6-R^7)_a-N^8R^9$ in which $R^5$ and $R^7$ are independently a divalent hydrocarbon group having 1-6 carbon atoms, $R^6$, $R^8$ and $R^9$ are independently a hydrogen atom or a monovalent hydrocarbon group having 1-20 carbon atoms, and a is a number of 0-3, wherein the molar ratio (1):(2):(3) of the structural units (1), (2) and (3) is 1:0.001-0.1:0.001-0.1, and at least one terminal of the terpolymer is capped with a hydroxyl group; and

(B) one or more cationic non-silicone conditioning components.

The hair cosmetic composition according to the present invention can impart smoothness, softness and antistatic effect to hair, permits good finish that the hair becomes soft, moist and innocent of oil or not sticky to the touch, makes the hair hard to become disheveled hair during sleep and hence has excellent retention of hairstyle set, has high remaining ability on the hair, so that it can fully exhibit its effects even on hair damaged by permanent waving, blow-dry and/or the like, and also possesses excellent stability.

The above and other objects, features and advantages of the present invention will become apparent from the following description and the appended claims.

DETAILED DESCRIPTION OF THE PREFERRED EMBODIMENTS

The amino-modified terpolymer of the component (A) useful in the practice of the present invention has structural units represented by the formulae (1), (2) and (3) (hereinafter referred to as the structural units (1), (2) and (3), respectively). In the formulae, examples of the monovalent hydrocarbon groups having 1-20 carbon atoms include alkyl groups such as methyl, ethyl, propyl, butyl, octyl, lauryl and stearyl groups; alkenyl groups such as vinyl and allyl groups; aryl groups such as phenyl, tolyl and naphthyl groups; aralkyl groups such as a 2-phenylethyl group; cycloalkyl groups such as cyclopentyl and cyclohexyl groups; and substituted groups thereof, in which hydrogen atoms bonded to carbon atoms of the above-mentioned groups are partially or entirely substituted by their corresponding number of substituents such as halogen atoms (for example, fluorine atom, chlorine atom, bromine atom, etc.), and cyano and mercapto groups, for example, 3,3,3-trifluoropropyl, cyanopropyl and mercaptopropyl groups. Of these, those having 1-6 carbon atoms, particularly, methyl, ethyl, propyl and phenyl group are preferred, with methyl and phenyl groups being further preferred.

Examples of the monovalent hydrocarbon groups having 1-6 carbon atoms include those having 1-6 carbon atoms among the above.

Examples of the divalent hydrocarbon groups having 1-6 carbon atoms include alkylene groups such as methylene, ethylene, trimethylene, tetramethylene and hexamethylene groups; a phenylene group; and the like. Of these, ethylene and trimethylene groups are particularly preferred.

Preferable examples of the structural unit (1) include $[(CH_3)_2SiO]$, $[(CH_3)(C_6H_5)SiO]$, $[(C_6H_5)_2SiO]$, $[(C_2H_5)_2SiO]$, $[(CH_3)(C_2H_5)SiO]$, $[(C_3H_7)_2SiO]$ and $[(CH_3)(C_3H_7)SiO]$.

Preferable examples of the structural unit (2) include $[(CH_3)(H_2NC_2H_4NHC_3H_6)SiO]$ and $[(CH_3)(H_2NC_3H_6)SiO]$.

Preferable examples of the structural unit (3) include $[(CH_3)SiO_{3/2}]$, $[(C_6H_5)SiO_{3/2}]$,$[(C_2H_5)SiO_{3/2}]$ and $[(C_3H_7)_2SiO_{3/2}]$.

The molar ratio (1):(2):(3) of these structural units must be 1:0.001-0.1:0.001-0.1. The molar ratio is preferably 1:0.01-0.05:0.01-0.05, most preferably 1:0.015-0.035:0.015-0.035. If the molar ratio is outside the above range, for

example, the structural units (2) and (3) are less than the lower limit of the above range, the resulting hair cosmetic composition is lowered in remaining ability on hair and can impart only insufficient smoothness to hair. On the other hand, if the structural units (2) and (3) are more than the upper limit of the above range, the resulting hair cosmetic composition can impart only insufficient softness to hair. It is not hence preferable to contain the structural units (2) and (3) in any proportions outside the above range.

In the amino-modified terpolymer containing these structural units, it is necessary for at least one terminal of the terpolymer to be capped with a hydroxyl group in order to improve its film-forming property. It is also preferred that the terminal other than the terminal capped with the hydroxyl group be terminated by an alkoxy group.

In the amino-modified terpolymer of the component (A), it is only necessary to contain the structural units (1), (2) and (3). No limitation is imposed on the bonding order thereof, and the like. For example, they may be bonded in the form of blocks or at random. The terpolymer may also contain structural units other than these structural units.

It is preferable to use, as the component (A), an amino-modified terpolymer emulsion obtained by emulsion polymerization because the emulsion is excellent in dispersibility in any water-based hair cosmetic composition.

The amino-modified terpolymer of the component (A) can be prepared in accordance with a method known per se in the art using suitable organosilanes or organopolysiloxanes which may be converted to an organopolysiloxane having the structural units (1), (2) and (3) through polymerization, condensation reaction and/or the like. Following the process described in, for example, Japanese Patent Publication No. 66896/1992, an organopolysiloxane represented by the following general formula (4) or (5), an amino-modified organopolysiloxane or amino-modified organosilane represented by the following general formula (6), (7) or (8) and an organosilane represented by the following general formula (9) or a partially hydrogenated condensate thereof are dispersed in water in presence of a cationic surfactant or nonionic surfactant, and then emulsion-polymerized in the presence of a alkaline catalyst, after which the reaction mixture is neutralized, thereby preparing the terpolymer.

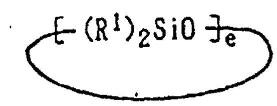 (4)

$$R^{11}O—[(R^1)_2SiO]_f—R^{11} \quad (5)$$

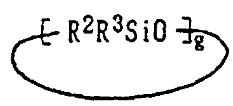 (6)

$$R^{12}O—[R^2R^3SiO]_h—R^{12} \quad (7)$$

$$R^2R^3Si(OR^{13})_2 \quad (8)$$

$$R^4Si(OR^{14})_3 \quad (9)$$

wherein $R^{11}$, $R^{12}$, $R^{13}$ and $R^{14}$ may be identical with or different from one another and are independently a hydrogen atom or a hydrocarbon group having 1-6 carbon atoms, e and g are independently a number of 3-10, f is a number of 1-1000, h is a number of 2-100, and $R^1$, $R^2$, $R^3$ and $R^4$ have the same meaning as defined above.

When these compounds are dispersed in water, a cationic surfactant and a nonionic surfactant are preferably used in combination with such compounds in order to improve the emulsifying state of the system and satisfy a variety of stability. No particular limitation is imposed on the cationic surfactant and nonionic surfactant used herein. However, examples of the cationic surfactant include alkyltrimethylammonium chlorides, alkylbenzylammonium chloride, dialkyldimethylammonium chloride and alkyltrimethylammonium bromides. Lauryltrimethylammonium chloride, cetyltrimethylammonium chloride, stearyltrimethylammonium chloride and the like are particularly preferred. It is preferred that the cationic surfactant be used in a proportion of 0.1-10 wt.%, particularly 1-5 wt.% based on the whole system because the stability of the resultant emulsion becomes excellent. On the other hand, examples of the nonionic surfactant include

polyoxyethylene alkyl ethers, polyoxyethylene alkylphenyl ethers, polyoxyethylene alkyl esters, sorbitan fatty acid esters, polyoxyethylene sorbitan fatty acid esters and sucrose fatty acid esters. Polyoxyethylene lauryl ether, polyoxyethylene myristyl ether, polyoxyethylene cetyl ether, polyoxyethylene tridecyl ether, polyoxyethylene oleyl ether, polyoxyethylene stearyl ether and the like are particularly preferred. The number of moles of ethylene oxide added is preferably 3-50 moles on the average. It is preferred that the nonionic surfactant be used in a proportion of 0.1-5 wt.%, particularly 0.5-2 wt.% based on the whole system because the stability of the resultant emulsion becomes excellent.

Examples of the alkaline catalyst used in the subsequent emulsion polymerization include sodium hydroxide, potassium hydroxide, rubidium hydroxide, sodium carbonate and tetraalkylammonium hydroxides. No particular limitation is imposed on the amount of these alkaline catalysts to be used. It is however preferable to use them in a range of 0.1-10 parts by weight per 100 parts by weight of the organopolysiloxane represented by the general formula (4) or (5).

With respect to the emulsion polymerization, it is only necessary to add the alkaline catalyst to the emulsified dispersion obtained in the preceding process and proceed with polymerization for from several hours to about 1 week at a proper temperature of 20-80°C while stirring the system.

After completion of the polymerization, the reaction mixture is neutralized with an organic or inorganic acidic substance such as, for example, hydrochloric acid, sulfuric acid, phosphoric acid, formic acid, acetic acid, citric acid, lactic acid or glycolic acid.

The amino-modified terpolymer thus obtained exhibits a nonfluidic gel-like appearance after evaporating water therefrom.

For the emulsion, it is industrially preferable to use one containing, as solids, the amino-modified terpolymer in a proportion of 1-90 wt.%, preferably 10-70 wt.%, more preferably 20-50 wt.%.

The amino-modified terpolymer of the component (A) is preferably incorporated in a proportion of 0.001-10 wt.% of the whole composition. From an economical point of view, it is particularly preferable to incorporate the terpolymer in a proportion of 0.01-5 wt.%, most preferably 0.1-2 wt.%.

Example of the cationic non-silicone conditioning component of the component (B) include cationic surfactants such as quaternary ammonium salts; and water-soluble cationic polymers.

Of these, the cationic surfactants include, for example, the following quaternary ammonium salts:

(B-1) Quaternary ammonium salts represented by the general formula (10):

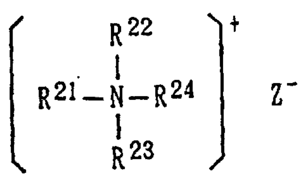 (10)

wherein at least one of $R^{21}$, $R^{22}$, $R^{23}$ and $R^{24}$ is an alkyl or alkenyl group which has 8-28 carbon atoms in total and may be substituted by an alkoxy, alkenyloxy, alkanoylamino or alkenoylamino group, the remainder are independently a benzyl group or an alkyl or hydroxyalkyl group having 1-5 carbon atoms, and $Z^-$ is a halide ion or organic anion.

Among those represented by the general formula (10), preferable examples include branched quaternary ammonium salts represented by the following general formulae (11) to (13):

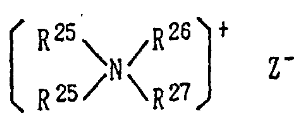 (11)

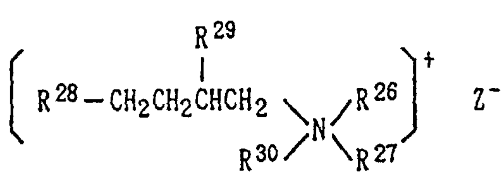 (12)

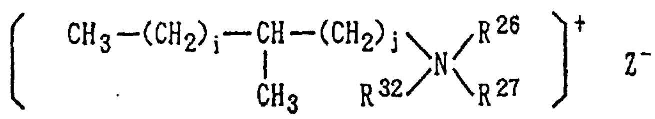 (1 3)

wherein $R^{25}$ is a group consisting of a mixture of (a) a branched alkyl group represented by

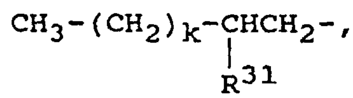

in which $R^{31}$ is a methyl or ethyl group, and k is such an integer that the total number of carbon atoms in the alkyl group amounts to 8-16, and (b) a linear alkyl group represented by $CH_3$-$(CH_2)_l$-, in which l is an integer of 7-15, the branching rate (a)/(a) + (b) of said mixture being 10-100%, $R^{26}$ and $R^{27}$ are independently a benzyl group, or an alkyl or hydroxyalkyl group having 1-3 carbon atoms, $R^{28}$ and $R^{29}$ are independently an alkyl group having 2-12 carbon atom, $R^{30}$ is a group

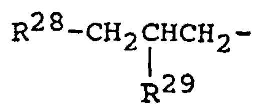

or an alkyl group having 1-3 carbon atoms, $R^{32}$ is a group

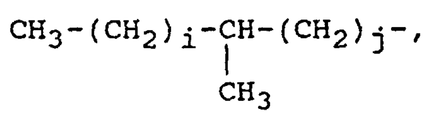

in which i is an integer of 2-14, and j is an integer of 3-11, with the proviso that the sum of i and j amounts to 9-21, or an alkyl group having 1-3 carbon atom, and $Z^-$ is a halide ion or organic anion.

The branched quaternary ammonium salts represented by the general formula (11) are generally synthesized, for example, by using, as a raw material, an oxo alcohol having 8-16 carbon atoms. Examples thereof include dialkyldimethylammonium salts, dialkylmethylhydroxyethylammonium salts and dialkylmethylbenzylammonium salts, which all have alkyl groups derived from the oxo alcohol.

In the present invention, the branched quaternary ammonium salts in which the branching rate of the $R^{25}$ in the formula (11) is generally 10-100% are used. Those having a branching rate of 10-50% are preferred. Besides, the branched quaternary ammonium salts in which the total number of carbon atoms in $R^{25}$ is 8-16 are used. Those having a specific distribution of number of carbon atoms are preferred, with those having the following distribution being particularly preferred.

$C_8$-$C_{11}$: 5% or lower;
$C_{12}$: 10-35%;
$C_{13}$: 15-40%;
$C_{14}$: 20-45%;
$C_{15}$: 5-30%; and
$C_{16}$: 5% or lower.

Specific examples of such branched quaternary ammonium salts include dialkyldimethylammonium chlorides which have alkyl groups having 8-16 carbon atoms and a branching rate of 10-50%.

Besides, the branched quaternary ammonium salts represented by the general formula (12) are generally synthesized by using, as a raw material, Guerbet alcohol,

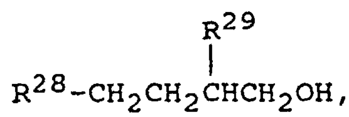

having 8-28 carbon atoms. Preferable examples thereof include alkyltrimethylammonium salts, alkyldimethylbenzylammonium salts, dialkyldimethylammonium salts, dialkylmethylhydroxyethylammonium salts and dialkylmethylbenzylammonium salts, which all have alkyl group(s) derived from the Guerbet alcohol. Of these, particularly preferable, specific examples include 2-decyltetradecyltrimethylammonium chloride, 2-dodecylhexadecyltrimethylammonium chloride, di-2-hexyldecyldimethylammonium chloride and di-2-octyldodecyldimethylammonium chloride.

Preferable examples of the methyl-branched quaternary ammonium salts represented by the general formula (13) include those in which the sum of i and j amounts to 15.

Specific examples of Z, which is a counter ion to each of the quaternary ammonium salts represented by the general formulae (10), (11), (12) and (13), include halide ions such as chloride, iodide and bromide ions; and organic anions such as methosulfates, ethosulfates, methophosphates and ethophosphates.

(B-2) Quaternary ammonium salt represented by the general formula (14):

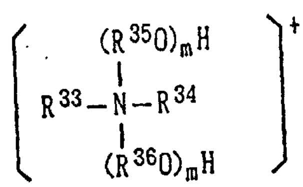 (14)

wherein at least one of $R^{33}$ and $R^{34}$ is an alkyl or alkenyl group which has 8-28 carbon atoms in total and may be substituted by an alkoxy, alkenyloxy, alkanoylamino or alkenoylamino group, the remainder is a benzyl group or an alkyl or hydroxyalkyl group having 1-5 carbon atoms, $R^{35}$ and $R^{36}$ are independently an alkylene group having 2-3 carbon atoms, $Z^-$ is a halide ion or organic anion, and each m may be identical or different and is independently an integer of 1-20.

(B-3) Quaternary ammonium salts represented by the general formula (15):

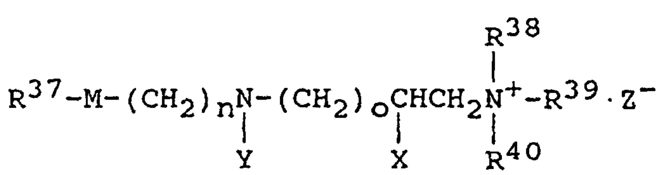 (15)

wherein $R^{37}$ is a linear or branched alkyl or alkenyl group having 7-21 carbon atoms, $R^{38}$, $R^{39}$ and $R^{40}$ are independently an alkyl or hydroxyalkyl group having 1-4 carbon atoms, M is -CONG-, in which G is a hydrogen atom, or an alkyl or hydroxyalkyl group having 1-3 carbon atoms, -O- or -COO-, X is a hydrogen atom or hydroxyl group, $Z^-$ is a halide ion or organic anion, Y is a hydrogen atom, an alkyl or hydroxyalkyl group having 1-3 carbon atoms, or a group represented by the formula (16):

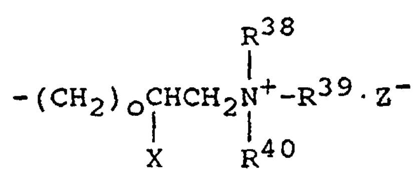 (16)

in which $R^{38}$, $R^{39}$, $R^{40}$, X and $Z^-$ have the same meaning as defined above, with the proviso that when G is an alkyl or hydroxyalkyl group having 1-3 carbon atoms, Y is not an alkyl or hydroxyalkyl group having 1-3 carbon atoms, n is 2 or 3, and o is an integer of 0-5, with the proviso that when o is 1, X is a hydrogen atom or hydroxyl

group, and when o is 0, 2, 3, 4 or 5, X is a hydrogen atom.

Among those represented by the general formula (15), those represented by the following general formula (17):

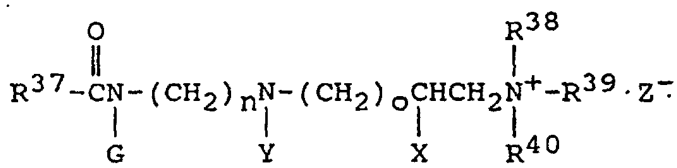

(17)

wherein $R^{37}$, $R^{38}$, $R^{39}$, $R^{40}$, G, X, Y, $Z^-$, n and o have the same meaning as defined above, are preferred, with those represented by the following general formula (18):

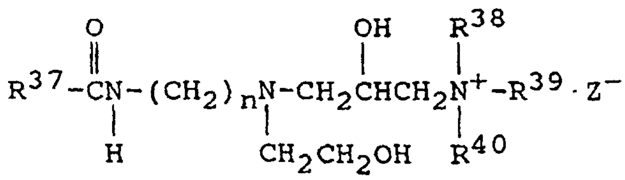

(18)

wherein $R^{37}$, $R^{38}$, $R^{39}$, $R^{40}$ and $Z^-$ have the same meaning as defined above, being particularly preferred.

(B-4) Asymmetric quaternary ammonium salts represented by the general formula (19):

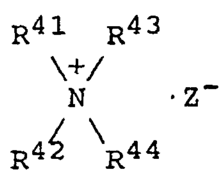

(19)

wherein $R^{41}$ is an alkyl group having a branched chain and 8-28 carbon atoms in total, $R^{42}$ is a linear alkyl or alkenyl group having 8-22 carbon atoms, for example, dodecyl, octadecyl or docosyl, $R^{43}$ and $R^{44}$ are independently an alkyl group having 1-4 carbon atoms or a hydrogen atom, with the proviso that $R^{43}$ and $R^{44}$ are not hydrogen atoms at the same time, and $Z^-$ has the same meaning as defined above.

Here, the branched alkyl group indicated by $R^{41}$ is a group derived from a branched alcohol as a raw material. Examples thereof include alkyl groups derived from Guerbet alcohol and oxo alcohol, such as 2-(3-methylhexyl)-7-methyl-1-decyl, 2-(1-methyl-3,3-dimethylbutyl)-5-methyl-7,7-dimethyloctyl and 2-hexyl-1-decyl.

The Guerbet alcohol means an alcohol represented by the formula (20):

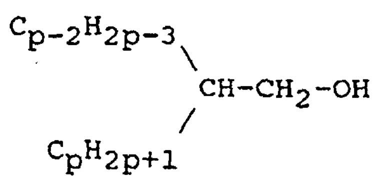

(20)

wherein p is an integer of 4-14. On the other hand, the oxo alcohol is a generic name for alcohols obtained by an oxo process making use of an α-olefin as a raw material, and derivatives thereof, and includes, for example, alcohols represented by the formulae (21) and (22):

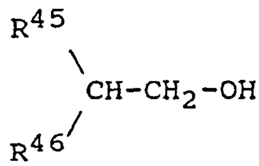

(21)

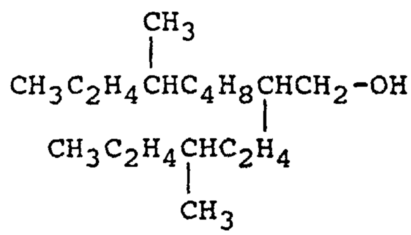

(22)

wherein $R^{45}$ is an alkyl group having 1-10 carbon atoms, $R^{46}$ is an alkyl group having 5-10 carbon atoms, with the proviso that the total number of carbon atoms of $R^{45}$ and $R^{46}$ is 10-18.

Specific examples of these alcohols include Fine Oxocol 140, Fine Oxocol 1600, Fine Oxocol 180, Fine Oxocol 180N, Fine Oxocol 1800, Fine Oxocol 2000 and Fine Oxocol 2600 (all, products of Nissan Chemical Industries, Ltd.), Diadol 18G (product of Mitsubishi Chemical Industries Limited), Dobanol 23-1 (product of Mitsubishi Petrochemical Company, Limited), EXXAL 18 and EXXAL 20 (both, products of Exxon Chemical Japan Ltd.) and Emasol 871 (product of Emery Industries, Inc.). Isostearyl alcohol obtained by methyl-esterifying Emasol 871 and then reducing the resultant ester is also included.

Among those represented by the general formula (19), preferable examples include N-2-(3-methylhexyl)-7-methyl-1-decyl-N-dodecyl-N,N-dimethylammonium chloride, N-2-(3-methylhexyl)-7-methyl-1-decyl-N-octyl-N,N-dimethylammonium chloride, N-2-(3-methylhexyl)-7-methyl-1-decyl-N-octadecyl-N,N-dimethylammonium chloride, N-2-hexyl-1-decyl-N-dodecyl-N,N-dimethylammonium chloride, and those represented by the formula (23):

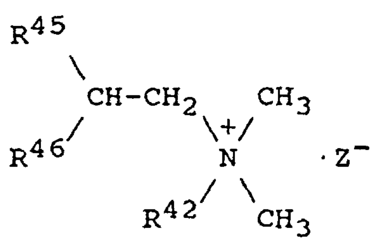

(23)

wherein $R^{42}$, $R^{45}$, $R^{46}$ and $Z^-$ have the same meaning as defined above.

Among these cationic surfactants, the asymmetric quaternary ammonium salts represented by the general formula (19) are particularly preferred. Further, those in which the branched alkyl group indicated by $R^{41}$ in the general formula (19) is an alkyl group derived from Fine Oxocol 140, Fine Oxocol 1600, Fine Oxocol 180N or Fine Oxocol 1800, or Diadol 18G, $R^{42}$ is a dodecyl or octadecyl group, $R^{43}$ and $R^{44}$ are methyl groups, and $Z^-$ is a chloride ion are preferred.

These cationic surfactants of the component (B) may be used either singly or in any combination thereof and are preferably incorporated in a proportion of 0.005-20 wt.% of the whole composition. From the viewpoint of economy and smell, it is particularly preferable to incorporate them in a proportion of 0.01-10 wt.%, most preferably 0.1-5 wt.%.

Among the cationic non-silicone conditioners of the component (B), the water-soluble cationic polymers are water-soluble polymers containing amino or ammonium groups bonded to their polymer chains, or comprising dimethyldiallylammonium halides as structural units. The polymers may partially contain anionic, nonionic and/or amphoteric structural units. Examples thereof include cationized cellulose derivatives, cationic starch, cationized guar gum derivatives, diallyl quaternary ammonium salt polymers, diallyl quaternary ammonium salt/acrylamide copolymers, diallyl quaternary ammonium salt/acrylic acid copolymers and quaternized poly(vinyl pyrrolidone) derivatives.

Preferred as the cationized cellulose derivatives are, for examples, those represented by the following general formula (24):

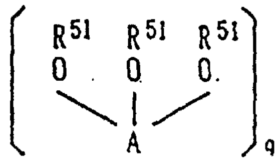

(2 4)

wherein A is a residue of an anhydroglucose unit, q is an integer of 50-20,000, and each $R^{51}$ is independently a substituent group represented by the following general formula (25):

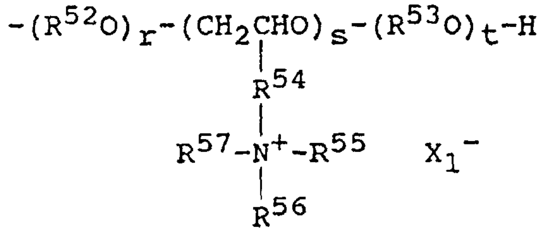

(25)

in which $R^{52}$ and $R^{53}$ are independently an alkylene group having 2-3 carbon atoms, r is an integer of 0-10, s is an integer of 0-3, t is an integer of 0-10, $R^{54}$ is an alkylene or hydroxyalkylene group having 1-3 carbon atoms, $R^{55}$, $R^{56}$ and $^{57}$ may be identical with or different from one another and are independently an alkyl, aryl or aralkyl group having 1-18 carbon atom, or may form a heterocyclic ring together with the nitrogen atom contained in the formula, and $X_1^-$ is an anion (chloride, bromide, iodide, sulfate, sulfonate, methylsulfate, phosphate, nitrate or the like).

The degree of substitution of the cations in these cationized cellulose derivatives is preferably 0.01-1, namely, the average value of s per anhydroglucose unit is preferably 0.01-1, more preferably 0.02-0.5. The sum of r and T is 1-3 on the average. Any degree of substitution of the cations lower than 0.01 is insufficient. The degree of substitution may exceed 1. However, it is preferably not higher than 1 from the viewpoint of yield. The molecular weight of the cationized cellulose derivative used herein is preferably within a range of 100,000-3,000,000.

Preferred as the cationic starch are those represented by the following general formula (26):

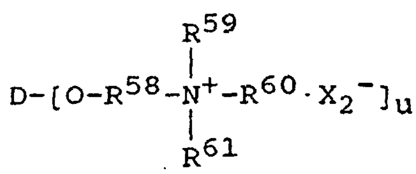

(26)

wherein D is a residue of starch, $R^{58}$ is an alkylene or hydroxyalkylene group, $R^{59}$, $R^{60}$ and $R^{61}$ are independently an alkyl, aryl or aralkyl group having 1-10 carbon atoms or may form a heterocyclic ring together with the nitrogen atom contained in the formula, $X_2^-$ is an anion (chloride, bromide, iodide, sulfate, sulfonate, methylsulfate, phosphate, nitrate or the like), and u is a positive integer.

The degree of substitution of the cations in these cationic starch derivatives is preferably 0.01-1, namely, the cationic groups are preferably introduced in a proportion of 0.01-1 group, more preferably 0.02-0.5 group per anhydroglucose unit. Any degree of substitution of the cations lower than 0.01 is insufficient. The degree of substitution may exceed 1. However, it is preferably not higher than 1 from the viewpoint of yield.

Preferred as the cationized guar gum derivatives are those represented by the following general formula (27):

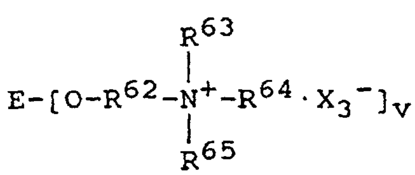

(27)

wherein E is a residue of guar gum, $R^{62}$ is an alkylene or hydroxyalkylene group, $R^{63}$, $R^{64}$ and $R^{65}$ are independently

an alkyl, aryl or aralkyl group having 1-10 carbon atoms or may form a heterocyclic ring together with the nitrogen atom contained in the formula, $X_3^-$ is an anion (chloride, bromide, iodide, sulfate, sulfonate, methylsulfate, phosphate, nitrate or the like), and v is a positive integer.

The degree of substitution of the cations in these cationized guar gum derivatives is preferably 0.01-1, namely, the cationic groups are preferably introduced in a proportion of 0.01-1 group, more preferably 0.02-0.5 group per sugar unit. Cationic polymers of this type are described in Japanese Patent Publication Nos. 35640/1983 and 46158/1985 and Japanese Patent Application Laid-Open No. 53996/1983, and are commercially available under the trade name of Jaguar (product of Celanese Schtein Hall Co.).

Preferred as the cationic diallyl quaternary ammonium salt polymers and diallyl quaternary ammonium salt/acrylamide copolymers are those represented by the following general formula (28) or (29):

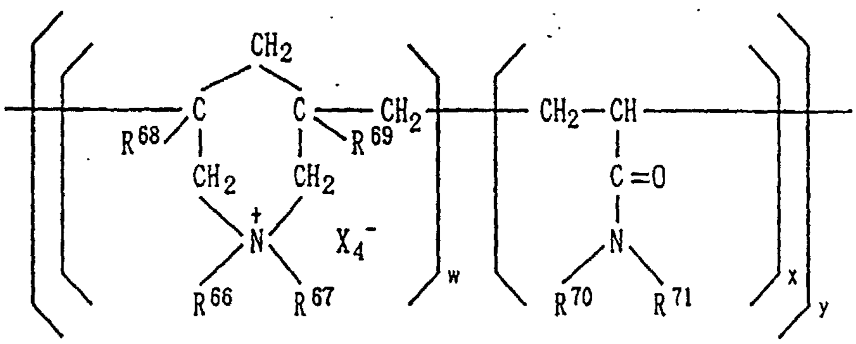

(28)

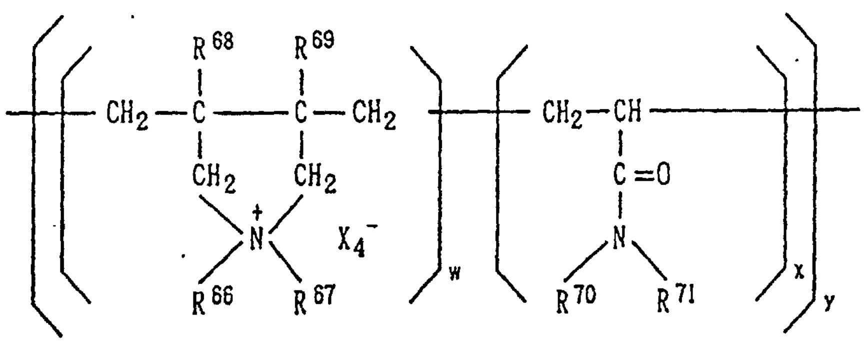

(29)

wherein $R^{66}$ and $R^{67}$ may be identical with or different from each other and are independently a hydrogen atom, or an alkyl (having 1-18 carbon atoms), phenyl, aryl, hydroxyalkyl, amidoalkyl, cyanoalkyl, alkoxyalkyl or carboalkoxyalkyl group, $R^{68}$, $R^{69}$, $R^{70}$ and $R^{71}$ may be identical with or different from one another and are independently a hydrogen atom, or an alkyl (having 1-3 carbon atoms) or phenyl group, $X_4^-$ is an anion (chloride, bromide, iodide, sulfate, sulfonate, methylsulfate, phosphate, nitrate or the like), w is an integer of 1-50, x is an integer of 0-50, and y is an integer of 150-8,000.

These diallyl quaternary ammonium salt polymers or diallyl quaternary ammonium salt/acrylamide copolymers preferably have a molecular weight within a range of about 30,000-10,000,000, more preferably 100,000-5,000,000.

These diallyl quaternary ammonium salt polymers or diallyl quaternary ammonium salt/acrylamide copolymers may contain anionic, nonionic and/or amphoteric structural units other than these structural units. For example, diallyl quaternary ammonium salt/acrylic acid/acrylamide copolymers, diallyl quaternary ammonium salt/acrylic acid copolymer and the like may also be used. Examples of commercial products thereof include Mercoat 100, Mercoat 550, Mercoat S, Mercoat 2200, Mercoat 280, Mercoat 295 and Mercoat 3330 (all, product of Calgon Corp.)

Preferred as the quaternized poly(vinyl pyrrolidone) derivatives are those represented by the following general formula (30):

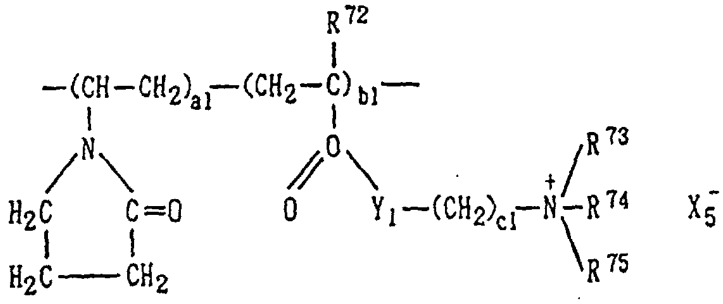

(3 0)

wherein $R^{72}$ is a hydrogen atom or an alkyl group having 1-3 carbon atoms, $R^{73}$, $R^{74}$ and $R^{75}$ may be identical with or different from one another and are independently a hydrogen atom, or an alkyl, hydroxyalkyl, amidoalkyl, cyanoalkyl, alkoxyalkyl or carboalkoxyalkyl having 1-4 carbon atoms, $Y_1$ is an oxygen atom or an NH group in an amide bond, $X_5^-$ is an anion (chloride, bromide, iodide, sulfate, sulfonate, alkylsulfate having 1-4 carbon atoms, phosphate, nitrate or the like), c1 is an integer of 1-10, a1 plus b1 is an integer of 20-8,000.

These quaternized poly(vinyl pyrrolidone) derivatives preferably have a molecular weight within a range of 10,000-2,000,000, more preferably 50,000-1,500,000.

Among the components (B), these cationic polymers may be used either singly or in any combination thereof and are preferably incorporated in a proportion of 0.001-10 wt.% of the whole composition. From the viewpoint of economy and smell, it is particularly preferable to incorporate it in a proportion of 0.1-5 wt.%, most , preferably 0.1-2 wt.%.

The component (A) and the component (B) are preferably incorporated in a weight ratio of 1/100 to 100/1, more preferably 1/50 to 50/1, most preferably 1/20 to 20/1.

The hair cosmetic compositions according to the present invention may comprise silicone derivatives (C) other than the component (A) in addition to the essential components described above. This incorporation can synergistically more enhance the function of the silicones.

Illustrated as such silicone derivatives (C) are those described in the following 1) to 9):

1) Dimethyl polysiloxanes:

$$(CH_3)_3SiO[(CH_3)_2SiO]_{d1}Si(CH_3)_3$$

(d1 = 3-20,000)

2) Methylphenyl polysiloxanes:

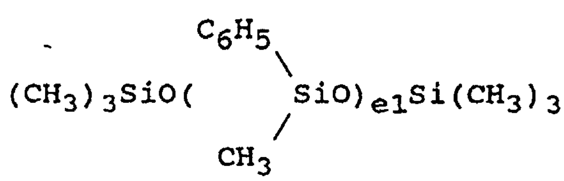

(e1 = 1-20,000)

$$(CH_3)_3SiO[(CH_3)_2SiO]_{f1}C(C_6H_5)_2SiO_3]_{g1}Si(CH_3)_3$$

(d1 + g1 = 1-500)

3) Polyether-modified silicones:

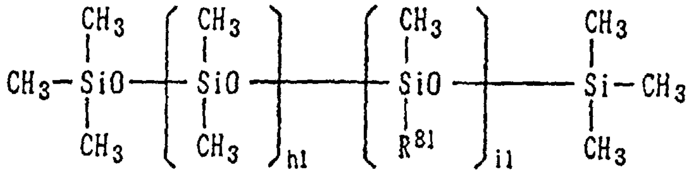

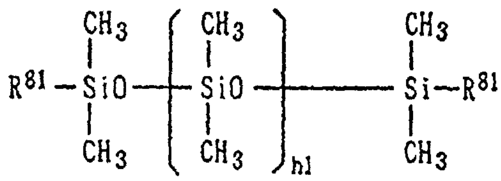

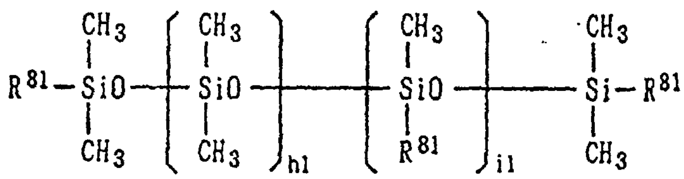

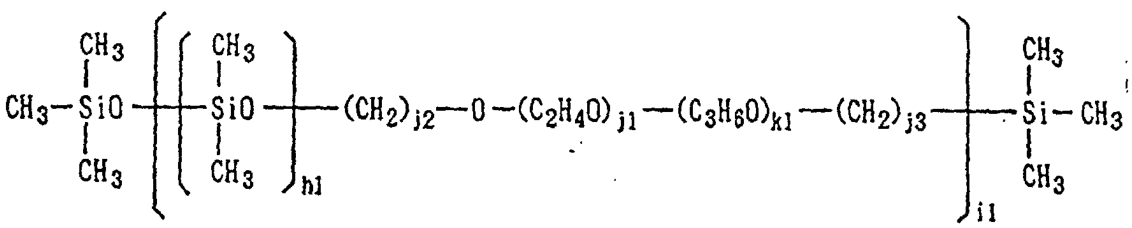

wherein $R^{81}$ is $-(CH_2)_3-O-(C_2H_4O)_{j1}-(C_3H_6O)_{k1}-G$, in which G is an alkyl group having 1-12 carbon atom or a hydrogen atom, j1 is a number of 0-50, and k1 is a number of 0-50 (j1 + k1 > 1), j2 and j3 are independently a number of 0-10, h1 is a number of 1-2000, and i1 is a number of 1-1000.

4) Epoxy-modified silicones:

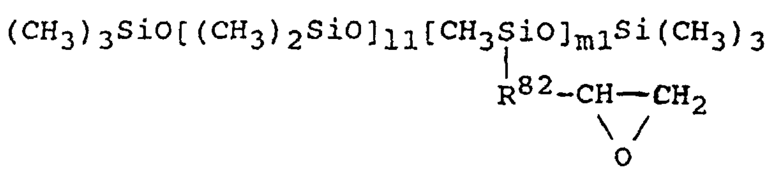

wherein $R^{82}$ is an alkylene group having 1-3 carbon atoms, 11 is a number of 1-500 (preferably 1-250), and m1 is a number of 1-50 (preferably 1-30).

5) Fluorine-modified silicones:

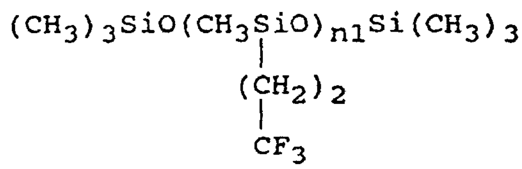

wherein n1 is a number of 1-400 (preferably 1-250).

6) Alcohol-modified silicones:

$$HO(CH_2)\text{-}R^{83}\text{-}[(CH_3)_2SiO]_{o1}[(CH_3)_2SiR^{83}\text{-}CH_2OH$$

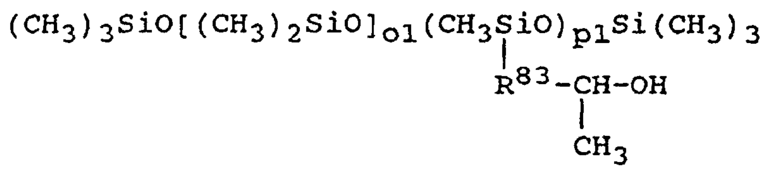

wherein $R^{83}$ is $C_zH_{2z}$ (2 = 0-4), and o1 and p1 are independently a number of 1-500 (preferably 1-200).

7) Alkyl-modified silicones:

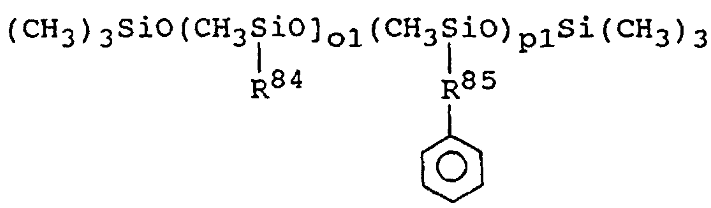

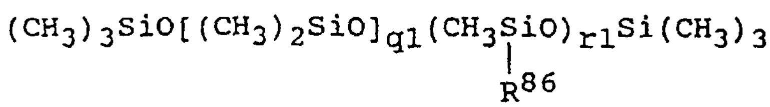

wherein $R^{84}$ is an alkyl group having 2-18 carbon atoms, $R^{85}$ is $C_zH_{2z}$ (z = 0-4), $R^{86}$ is an alkyl group having 10-16 carbon atoms, and q1 and r1 are independently a number of 1-500 (preferably 1-200).

8) Alkoxy-modified silicones:

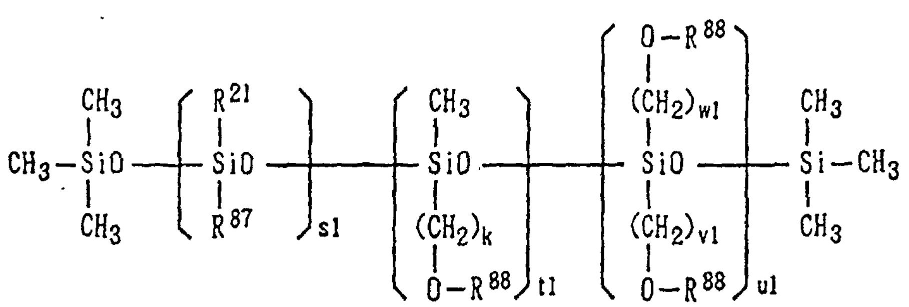

wherein $R^{87}$ is a methyl or phenyl group, each $R^{88}$ may be identical or different and is independently an alkyl group having 1-28 carbon atoms (preferably 12-22 carbon atoms), s1 is a number of 1-3000, t1 and u1 are independently such a number that t1 plus u1 amounts to 1-500, and k, v1 and w1 may be identical with or different from one another and are independently a number of 0-6.

9) Trimethylsiloxysilicic acids:

$$[(CH_3)_3SiO_{1/2}]_{x1}[SiO_2]_{y1}$$

wherein x1 is a number of 1-20, and y1 is a number of 0.1-50.

The manners of use of these silicone derivatives 1) to 9) include a case where they are used singly as it is, a case where at least two of them are premixed before use, and a case where they are used in the form of a microdispersion (silicone emulsion) in an aqueous medium in combination with one or more of surfactants routinely used as emulsifiers, for example, nonionic, anionic, cationic and amphoteric surfactants.

A silicone emulsion is obtained in accordance with an agitation and mixing process, an emulsion polymerization process or the like. As such silicone emulsions, for example, KM880, KM883, KM884, KM885, KM886, KM887, KM901, KM902, KM903, KM904, KM871P, Polon MF11C, Polon MF18 and KM4702 (all, products of Shin-Etsu Chemical Co., Ltd.), and the like are commercially available.

Among these silicone derivatives, dimethyl polysiloxanes and polyether-modified silicones are particularly preferred.

If used, these silicone derivatives are preferably incorporated in a proportion of 0.01-20 wt.%, particularly 0.1-10 wt.% of the whole composition.

In the hair cosmetic compositions according to the present invention, ingredients routinely used in hair cosmetic compositions, for example, pearl-like-hue-imparting agents, emulsifiers, perfume bases, coloring matter, antiseptics, antioxidants, thickeners, antidandruff agents, disinfectants, antiphlogistics, medicinally-effective agents such as vitamins, ultraviolet absorbents, and other ingredients described in Encyclopedia of Shampoo Ingredients (Micelle Press, 1985), may be suitably incorporated in addition to the above-described components within limits not impeding the effects of the inventive hair cosmetic compositions.

The hair cosmetic compositions according to the present invention can be formulated in accordance with methods known per se in the art, and may be widely applied to various hair cosmetic compositions such as hair shampoos, hair rinse-in-shampoos, hair rinses, hair treatment compositions, styling compositions, permanent wave compositions and hair dye compositions.

EXAMPLES:

The present invention will hereinafter be described in more detail by the following Examples. However, the present invention is not limited to these Examples. Prior to the description of the Examples, Synthetic Examples of the components (A) to be used in these Examples will be described.

Synthetic Example 1:

(Components)

| | | |
|---|---|---|
| (a) | Octamethylcyclotetrasiloxane | 400 g |
| (b) | Amino-modified organopolysiloxane represented by the following formula: | 15 g |

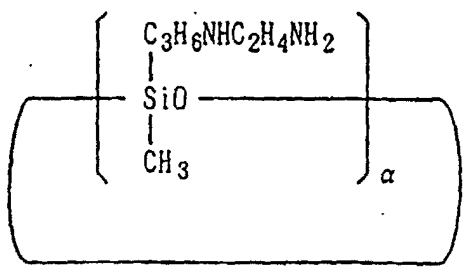

(α is an integer of 3-6)

| | | |
|---|---|---|
| (c) | Methyltriethoxysilane | 34 g |
| (d) | Cetyltrimethylammonium chloride (30% aqueous solution) | 100 g |
| | Polyoxyethylene (5) lauryl ether | 5 g |
| | Polyoxyethylene (20) lauryl ether | 5 g |
| (e) | Potassium hydroxide (5% aqueous solution) | 20 g |
| (f) | Acetic acid | 1 g |

(Preparation process)

The components (a), (b) and (c) were poured into a 2-liter glass beaker and intimately mixed by a homomixer into a solution. The component (d) and 532 g of water were then added to homogeneously emulsify and disperse the mixture. After the component (e) was then added to the resultant emulsion, the resulting mixture was heated at 70°C for 72 hours to conduct polymerization reaction. Thereafter, the component (f) was added to neutralize the reaction mixture, thereby obtaining an amino-modified terpolymer emulsion (A).

The thus-obtained amino-modified terpolymer contained structural units (1-1), (2-1) and (3-1) represented by the following formulae in a molar ratio of 1:0.017:0.035.

$$[(CH_3)_2SiO] \quad (1\text{-}1)$$

$$[(CH_3)(H_2NC_2H_4NHC_3H_6)SiO] \quad (2\text{-}1)$$

$$[(CH_3)SiO_{3/2}] \qquad (3\text{-}1).$$

The resultant emulsion was heated for 3 hours at 105°C to dry. As a result, it was found that residues after the drying were 43%, and nonfluidic white gel was formed. The emulsion thus obtained was excellent in stability as demonstrated by the fact that when left to stand in a thermostatic chamber controlled at 50°C, it did not separate even after 1 month.

Synthetic Example 2:

An amino-modified terpolymer emulsion (B) was obtained in the same manner as in Synthetic Example 1 except that the amounts of the components (b) and (c) in Synthetic Example 1 were changed to 20 g and 22 g, respectively.

The thus-obtained amino-modified terpolymer contained the structural units (1-1), (2-1) and (3-1) in a molar ratio of 1:0.023:0.023.

The resultant emulsion was heated for 3 hours at 105°C to dry. As a result, it was found that residues after the drying were 43%, and nonfluidic white gel was formed. The emulsion thus obtained was excellent in stability as demonstrated by the fact that when left to stand in a thermostatic chamber controlled at 50°C, it did not separate even after 1 month.

Synthetic Example 3:

An amino-modified terpolymer emulsion (C) was obtained in the same manner as in Synthetic Example 1 except that the amount of the component (c) in Synthetic Example 1 was changed to 22 g.

The thus-obtained amino-modified terpolymer contained the structural units (1-1), (2-1) and (3-1) in a molar ratio of 1:0.017:0.023.

The resultant emulsion was heated for 3 hours at 105°C to dry. As a result, it was found that residues after the drying were 42%, and nonfluidic white gel was formed. The emulsion thus obtained was excellent in stability as demonstrated by the fact that when left to stand in a thermostatic chamber controlled at 50°C, it did not separate even after 1 month.

Synthetic Example 4:

An amino-modified terpolymer emulsion (D) was obtained in the same manner as in Synthetic Example 1 except that the amounts of the components (b) and (c) in Synthetic Example 1 were changed to 0.7 g and 0.8 g, respectively.

The thus-obtained amino-modified terpolymer contained the structural units (1-1), (2-1) and (3-1) in a molar ratio of 1:0.0008:0.0008.

The resultant emulsion was heated for 3 hours at 105°C to dry. As a result, it was found that residues after the drying were 39%, and nonfluidic white gel was formed. The emulsion thus obtained was excellent in stability as demonstrated by the fact that when left to stand in a thermostatic chamber controlled at 50°C, it did not separate even after 1 month.

Synthetic Example 5:

An amino-modified terpolymer emulsion (E) was obtained in the same manner as in Synthetic Example 1 except that the amounts of the components (a), (b) and (c) in Synthetic Example 1 were changed to 300 g, 78 g and 87 g, respectively.

The thus-obtained amino-modified terpolymer contained the structural units (1-1), (2-1) and (3-1) in a molar ratio of 1:0.12:0.12.

The resultant emulsion was heated for 3 hours at 105°C to dry. As a result, it was found that residues after the drying were 44%, and a viscous, pale-yellow liquid having a viscosity of 1,200,000 cP at 25°C was formed.

Example 1:

Hair shampoos having their corresponding formulations shown in Table 1 were prepared and evaluated as to smoothness of running of fingers through hair upon use, softness of hair, moisturized feeling, antistatic property, no

stickiness to the touch, remaining ability on hair, resistance to becoming disheveled hair during sleep, retention of hairstyle set, and evenness of the feel against hair. The results are shown in Table 2.

(Preparation process)

Its corresponding components (1) to (5) and (9) shown in Table 1 were mixed and heated to 70°C, and the component (7) was added to the mixture into a solution, followed by cooling. The components (6) and (8) were then added at the time the temperature of the solution reached 50°C or lower, and the resultant mixture was uniformly mixed and kept at pH 6 with citric acid, thereby obtaining a hair shampoo.

(Evaluation methods)

(1) Smoothness of running of fingers through hair, softness of hair, moisturized feeling, antistatic property and no stickiness to the touch:

Each 20 g (15 cm long) of the hair of Japanese women, which had not been subjected to any hairdressing treatment such as cold permanent waving, was bound up to form a hair bundle N. On the other hand, each 20 g (15 cm long) of the hair of Japanese women, which had been subjected to cold permanent waving three times, was bound up to form a hair bundle P.

To these hair bundles, 1-2 g of each of the hair shampoos prepared above were applied to treat them in the conventional manner. Thereafter, the hair bundles were dried, and the intended items were organoleptically evaluated by an expert panel to rank in accordance with the following standards:

(Smoothness of running of fingers through hair)

A: Fingers were smoothly run through the hair;
B: Fingers were somewhat smoothly run through the hair;
C: Fingers were not smoothly run through the hair.

(Softness of hair)

A: The hair was very soft to the touch;
B: The hair was somewhat soft to the touch;
C: The hair was not soft at all to the touch.

(Moisturized feeling)

A: The hair felt moisturized;
B: The moisturized feeling was somewhat lacking;
C: The hair did not feel moisturized at all.

(Antistatic property)

A: No hair fly occurred when brushed;
B: Hair fly slightly occurred when brushed;
C: Hair fly occurred when brushed.

(No stickiness to the touch)

A: Not sticky to the touch;
B: Somewhat sticky to the touch;
C: Sticky to the touch.

(2) Remaining ability on hair:

Hair bundles treated with each of the hair shampoos in the same manner as described above were washed repeatedly three times with a plain shampoo consisting of 15 wt.% of sodium poly(oxyethylene) (3) lauryl ether sulfate, 3 wt.% of diethanolamide laurate and the balance of deionized water and kept at pH 7 with citric acid. Thereafter, the hair bundles were dried, and the remaining feeling on the hair was organoleptically evaluated to rank in accordance with the following standard:

A: A remaining feeling existed;
B: A remaining feeling slightly existed;
C: No remaining feeling existed.

(3) Resistance to becoming disheveled hair during sleep, retention of hairstyle set and evenness of the feel against hair:

After ten expert panelists used each of the hair shampoos for 2-3 days, the following items were organoleptically evaluated and rated as score +1 point where it was effective, score +0.5 point where it was somewhat effective or score 0 point where it was not effective. The items tested were ranked by the total point in accordance with the following standards. Incidentally, the ten expert panelists had the following hair styles and features:

Long straight hair: 2 panelists;
Long wavy hair: 2 panelists
Medium-length straight hair: 2 panelists;
Medium-length wavy hair: 2 panelists;
Short straight hair: 1 panelist; and
Short wavy hair: 1 panelist.

(Resistance to becoming disheveled hair during sleep)

A: Hard to become disheveled hair during sleep (score not lower than 6 points but not higher than 10 points);
B: Somewhat hard to become disheveled hair during sleep (score not lower than 3 points but lower than 6 points);
C: Easy to become disheveled hair during sleep (score not lower than 0 point but lower 3 points).

(Retention of hairstyle set)

A: The hairstyle lasted long (score not lower than 6 points but not higher than 10 points);
B: The hairstyle did not last much longer (score not lower than 3 points but lower than 6 points);
C: The hairstyle did not last (score not lower than 0 point but lower 3 points).

(Evenness of the feel against hair)

A: The feel against hair was even from the root to the tip of the hair (score not lower than 6 points but not higher than 10 points);
B: The feel against hair was somewhat even from the root to the tip of the hair (score not lower than 3 points but lower than 6 points);

C: The feel against hair was uneven between the root and the tip of the hair (score not lower than 0 point but lower 3 points).

Table 1

| | Component (wt.%) | Invention product | | | | | | Comparative product | | | | | |
|---|---|---|---|---|---|---|---|---|---|---|---|---|---|
| | | 1 | 2 | 3 | 4 | 5 | 6 | 1 | 2 | 3 | 4 | 5 | 6 |
| (1) | Sodium polyoxyethylene (3) lauryl sulfate | 5 | 5 | 5 | 5 | 5 | 5 | 5 | 5 | 5 | 5 | 5 | 5 |
| (2) | Disodium polyoxyethylene (7) lauryl sulfosuccinate | 5 | 5 | 5 | 5 | 5 | 5 | 5 | 5 | 5 | 5 | 5 | 5 |
| (3) | Decyl polyglucoside (average polymerization degree of glucose: 1.5) | 3 | 3 | 3 | 3 | 3 | 3 | 3 | 3 | 3 | 3 | 3 | 3 |
| (4) | N-Lauroyl-N'-carboxymethyl-N'-(2-hydroxyethyl)ethylene-diaminetriethanolamine | 3 | 3 | 3 | 3 | 3 | 3 | 3 | 3 | 3 | 3 | 3 | 3 |
| (5) | Diethanolamide laurate | 2 | 2 | 2 | 2 | 2 | 2 | 2 | 2 | 2 | 2 | 2 | 2 |
| (6) | Amino-modified terpolymer emulsion (Synthetic Ex. 1) | 2 | - | - | 2 | - | - | - | 2 | - | - | - | - |
| | Amino-modified terpolymer emulsion (Synthetic Ex. 2) | - | 2 | - | - | 2 | - | - | - | - | - | - | - |
| | Amino-modified terpolymer emulsion (Synthetic Ex. 3) | - | - | 2 | - | - | 2 | - | - | - | - | - | - |
| | Amino-modified terpolymer emulsion (Synthetic Ex. 4) | - | - | - | - | - | - | - | - | - | 2 | - | - |
| | Amino-modified terpolymer emulsion (Synthetic Ex. 5) | - | - | - | - | - | - | - | - | - | - | 2 | - |
| | Amodimethicone emulsion (SM8702C, product of Dow Corning Toray Silicone Co., Ltd.) | - | - | - | - | - | - | - | - | - | - | - | 2 |
| (7) | Cationized cellulose (Polymer JR400, product of UCC) | 1 | - | - | - | 1 | - | - | - | 1 | 1 | - | - |
| | Poly(diallyldimethylammonium chloride) (Mercoat 550 (product of Calgon Corp.) | - | 1 | - | - | - | 1 | - | - | - | - | 1 | 1 |
| | (Myristoyl-N-hydroxyethyl)aminoethyl-2-hydroxypropyltrimethylammonium chloride | - | - | 1 | 1 | - | - | - | - | - | - | - | - |
| (8) | Dimethyl polysiloxane (100,000 cP) | - | - | - | 1 | 1 | 1 | 1 | - | - | - | - | - |
| (9) | Purified water | Balance | | | | | | | | | | | |
| (10) | pH Adjustment (citric acid) | Amount sufficient to pH 6 | | | | | | | | | | | |

Table 2

| Evaluation result | Invention product | | | | | | Comparative product | | | | | |
|---|---|---|---|---|---|---|---|---|---|---|---|---|
| | 1 | 2 | 3 | 4 | 5 | 6 | 1 | 2 | 3 | 4 | 5 | 6 |
| (Smoothness of running of fingers through hair) | | | | | | | | | | | | |
| Hair bundle N | A | A | A | A | A | A | A | B | C | B | B | B |
| Hair bundle P | A | A | A | A | A | A | C | A | C | C | B | A |
| (Softness of hair) | | | | | | | | | | | | |
| Hair bundle N | A | A | A | A | A | A | C | C | B | C | B | B |
| Hair bundle P | A | A | A | A | A | A | C | B | C | C | B | B |
| (Moisturized feeling) | | | | | | | | | | | | |
| Hair bundle N | A | A | A | A | A | A | B | C | B | C | B | A |
| Hair bundle P | A | A | A | A | A | A | C | B | C | C | B | C |
| (Antistatic property) | | | | | | | | | | | | |
| Hair bundle N | A | A | A | A | A | A | C | C | B | C | B | A |
| Hair bundle P | A | A | A | A | A | A | C | B | B | B | C | B |
| (No stickiness to the touch) | | | | | | | | | | | | |
| Hair bundle N | A | A | A | A | A | A | B | A | B | A | A | A |
| Hair bundle P | A | A | A | A | A | A | A | A | A | A | A | A |
| (Remaining ability on hair) | | | | | | | | | | | | |
| Hair bundle N | A | A | A | A | A | A | B | C | C | C | B | B |
| Hair bundle P | A | A | A | A | A | A | C | A | C | C | B | B |
| (Resistance to becoming disheveled hair during sleep) | A | A | A | A | A | A | C | B | C | C | C | B |
| (Retention of hairstyle set) | A | A | A | A | A | A | C | B | C | C | B | B |
| (Evenness of the feel against hair) | B | B | B | A | A | A | C | C | C | C | B | C |

Example 2:

Hair rinses having their corresponding formulations shown in Table 3 were prepared, and their performance was evaluated in the same manner as in Example 1 except that a hair bundle (Hair bundle Q) obtained by binding up each 20 g (15 cm long) of the hair of Japanese women, which had not been subjected to any hairdressing treatment such as cold permanent waving and brushing the hair 1000 times, and a hair bundle (Hair bundle R) obtained by binding up each 20 g (15 cm long) of the hair of Japanese women, which had been subjected to bleaching treatment once, were used. The results are shown in Table 4.

(Preparation process)

Its corresponding component (6) shown in Table 3 was added to the component (7), and the mixture was heated to 70°C into a solution. The components (1), (4) and (5) were mixed and heated to 70°C into a solution. The latter solution was added to the former solution and thoroughly mixed, followed by addition of the components (2) and (3). The resultant mixture was cooled to room temperature with stirring, thereby obtaining a hair rinse.

Table 3

| Component (wt.%) | | Invention product | | | | | | Comp. product | | | |
|---|---|---|---|---|---|---|---|---|---|---|---|
| | | 7 | 8 | 9 | 10 | 11 | 12 | 7 | 8 | 9 | 10 |
| (1) | Stearyltrimethylammonium chloride | 1 | - | - | 1 | - | - | 1 | 1 | - | - |
| | 2-Dodecylhexadecyltrimethylammonium chloride | - | 1 | - | - | 1 | - | - | - | 1 | - |
| | Poly(diallyldimethylammonium chloride) (Mercoat 100 (product of Calgon Corp.) | - | - | 1 | - | - | 1 | - | - | - | 1 |
| (2) | Amino-modified terpolymer emulsion (Synthetic Ex. 1) | 1 | - | - | - | 1 | - | - | - | - | - |
| | Amino-modified terpolymer emulsion (Synthetic Ex. 2) | - | 1 | - | - | - | 4 | - | - | - | - |
| | Amino-modified terpolymer emulsion (Synthetic Ex. 3) | - | - | 4 | 1 | - | - | - | - | - | - |
| | Amino-modified terpolymer emulsion (Synthetic Ex. 4) | - | - | - | - | - | - | - | 1 | - | - |
| | Amino-modified terpolymer emulsion (Synthetic Ex. 5) | - | - | - | - | - | - | - | - | 1 | - |
| | Amodimethicone emulsion (SM8702C, product of Dow Corning Toray Silicone Co., Ltd.) | - | - | - | - | - | - | - | - | - | 4 |
| (3) | Dimethyl polysiloxane (70,000 cP) | - | - | - | 2 | 2 | 2 | 2 | - | - | - |
| (4) | Cetyl alcohol | 3 | 3 | - | 3 | 3 | - | 3 | 3 | 3 | - |
| (5) | Propylene glycol | 5 | 5 | 5 | 5 | 5 | 5 | 5 | 5 | 5 | 5 |
| (6) | Hydroxyethylcellulose | 1 | 1 | 1 | 1 | 1 | 1 | 1 | 1 | 1 | 1 |
| (7) | Purified water | Balance | | | | | | | | | |

Table 4

| Evaluation result | Invention product | | | | | | Comp. product | | | |
|---|---|---|---|---|---|---|---|---|---|---|
| | 7 | 8 | 9 | 10 | 11 | 12 | 7 | 8 | 9 | 10 |
| (Smoothness of running of fingers through hair) | | | | | | | | | | |
| Hair bundle Q | A | A | A | A | A | A | A | A | A | A |
| Hair bundle R | A | A | A | A | A | A | B | B | B | B |
| (Softness of hair) | | | | | | | | | | |
| Hair bundle Q | A | A | A | A | A | A | B | B | B | B |
| Hair bundle R | A | A | A | A | A | A | B | B | B | B |
| (Moisturized feeling) | | | | | | | | | | |
| Hair bundle Q | A | A | A | A | A | A | B | B | B | A |
| Hair bundle R | A | A | A | A | A | A | C | C | C | C |
| (Antistatic property) | | | | | | | | | | |
| Hair bundle Q | A | A | A | A | A | A | B | B | B | A |
| Hair bundle R | A | A | A | A | A | A | C | C | C | C |
| (No stickiness to the touch) | | | | | | | | | | |
| Hair bundle Q | A | A | A | A | A | A | A | A | A | A |
| Hair bundle R | A | A | A | A | A | A | A | A | A | A |

Table 4 (continued)

| Evaluation result | Invention product | | | | | | Comp. product | | | |
|---|---|---|---|---|---|---|---|---|---|---|
| | 7 | 8 | 9 | 10 | 11 | 12 | 7 | 8 | 9 | 10 |
| (Remaining ability on hair) | | | | | | | | | | |
| Hair bundle Q | A | A | A | A | A | A | A | A | B | A |
| Hair bundle R | A | A | A | A | A | A | C | B | C | B |
| (Resistance to becoming disheveled hair during sleep) | A | A | A | A | A | A | C | B | B | B |
| (Retention of hairstyle set) | A | A | A | A | A | A | C | B | C | B |
| (Evenness of the feel against hair) | B | B | B | A | A | A | C | B | C | C |

Example 3: (Hair rinse)

A hair rinse having the following formulation was prepared in accordance with the conventionally-known method.

The resultant hair rinse imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | N-(2-Decyl)tetradecyl-N,N,N-trimethylammonium chloride | 0.5 |
| (2) | Stearyltrimethylammonium chloride | 1.0 |
| (3) | Amino-modified terpolymer emulsion (Synthetic Example 1) | 15.0 |
| (4) | Lignoceric acid | 3.0 |
| (5) | Cetostearyl alcohol | 2.0 |
| (6) | Zinc pyrithione | 0.3 |
| (7) | Methylparaben | 0.2 |
| (8) | Perfume base | 0.4 |
| (9) | Deionized water | Balance |
| | Total | 100 |

Example 4: (Styling lotion)

A styling lotion having the following formulation was prepared in accordance with the conventionally-known method.

The resultant styling lotion imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | N-(2-Dodecyl)hexadecyl-N,N,N-trimethylammonium chloride | 0.7 |
| (2) | Polyethylene glycol | 0.5 |
| (3) | Amino-modified terpolymer emulsion (Synthetic Example 2) | 8.0 |
| (4) | Dimethyl polysiloxane (50,000 cP) | 0.5 |
| (5) | Liquid acrylic resin/liquid alkanolamine | 5.0 |
| (6) | Polyethylene glycol | 1.0 |
| (7) | Methacrylic ester polymer | 1.0 |
| (8) | Ethanol | 20.0 |
| (9) | Perfume base | 0.3 |
| (10) | Water | Balance |
| | Total | 100 |

Example 5: (Conditioning foam)

A conditioning foam having the following formulation was prepared in accordance with the conventionally-known method.

The resultant conditioning foam imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | N-(2-Decyl)tetradecyl-N,N,N-trimethylammonium chloride | 0.5 |
| (2) | Octyldodecyl myristate | 1.0 |
| (3) | Dipropylene glycol | 1.0 |
| (4) | Amino-modified terpolymer emulsion (Synthetic Example 3) | 5.0 |
| (5) | Polyether-modified silicone (KF6005, product of Shin-Etsu Chemical Co., Ltd.) | 0.5 |
| (6) | Glycerol | 2.5 |
| (7) | Liquid paraffin | 2.5 |
| (8) | One mole adduct of pentaerythritol isostearyl glycidyl ether | 0.5 |
| (9) | Ethanol | 5.0 |
| (10) | Methylparaben | 0.1 |
| (11) | Perfume base | 0.1 |
| (12) | Propellant (LPG) | 10.0 |
| (13) | Water | Balance |
| | Total | 100 |

Example 6: (First-package permanent wave composition)

A first-package permanent wave composition having the following formulation was prepared in accordance with the conventionally-known method.

The resultant first-package permanent wave composition imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | Ammonium thioglycolate | 6.0 |
| (2) | Amino-modified terpolymer emulsion (Synthetic Example 1) | 1.0 |
| (3) | Aqueous ammonia | 3.0 |
| (4) | Frost DS (disodium edetate) | 0.5 |
| (5) | Benzyl alcohol | 10.0 |
| (6) | N-(2-Decyl)tetradecyl-N,N,N-trimethylammonium chloride | 2.0 |
| (7) | Water | Balance |
| | Total | 100 |

Example 7: (Second-package permanent wave composition)

A second-package permanent wave composition having the following formulation was prepared in accordance with the conventionally-known method.

The resultant second-package permanent wave composition imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | Sodium bromate | 8.0 |

(continued)

| (Component) | | (wt.%) |
|---|---|---|
| (2) | N-(2-Dodecyl)hexadecyl-N,N,N-trimethylammonium chloride | 2.0 |
| (3) | Dimethyl polysiloxane (200 cSt) | 5.0 |
| (4) | Amino-modified terpolymer emulsion (Synthetic Example 1) | 0.5 |
| (5) | Water | Balance |
| | Total | 100 |

Example 8: (Hair shampoo):

A hair shampoo having the following formulation was prepared in accordance with the conventionally-known method.

The resultant hair shampoo imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | N-Lauroyl-N'-carboxymethyl-N'-(2-hydroxyethyl)ethylenediamine TBA salt | 10.0 |
| (2) | 18-Methyleicosanoic acid | 0.5 |
| (3) | Amino-modified terpolymer emulsion (Synthetic Example 2) | 5.0 |
| (4) | Disodium polyoxyethylene (5) lauryl sulfosuccinate | 5.0 |
| (5) | Diethanolamide laurate | 2.0 |
| (6) | Coconut oil fatty amide propylbetaine | 2.0 |
| (7) | Distearyldimethylammonium chloride | 0.1 |
| (8) | Cationized cellulose (Polymer JR400, product of UCC) | 0.15 |
| (9) | Perfume base | 0.5 |
| (10) | Coloring matter | q.s. |
| (11) | Water | Balance |
| | Total | 100 |

Example 9: (Hair treatment composition)

A hair treatment composition having the following formulation was prepared in accordance with the conventionally-known method.

The resultant hair treatment composition imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | 2-Dodecylhexadecyltrimethylammonium chloride | 1.5 |
| (2) | Stearyltrimethylammonium chloride | 2.0 |
| (3) | Cetostearyl alcohol | 3.0 |
| (4) | Oleic acid monoglyceride | 1.0 |
| (5) | Benzyl alcohol | 5.0 |
| (6) | Amino-modified terpolymer emulsion (Synthetic Example 3) | 3.0 |
| (7) | Hydroxyethylcellulose (1% aqueous solution, viscosity: 8,000 cP) | 0.5 |
| (8) | Methylparaben | 0.2 |
| (9) | Perfume base | 0.4 |
| (10) | Water | Balance |
| | Total | 100 |

Example 10: (Hair conditioning composition)

A hair conditioning composition having the following formulation was prepared in accordance with the conventionally-known method.

The resultant hair rinse imparted smoothness, softness and antistatic effect to hair, permitted good finish, made the hair hard to become disheveled hair during sleep and hence had excellent retention of hairstyle set, and possessed high remaining ability on the hair.

| (Component) | | (wt.%) |
|---|---|---|
| (1) | Di(2-hexadecyl)dimethylammonium chloride | 0.5 |
| (2) | Methylphenyl polysiloxane (300 cSt) | 1.0 |
| (3) | Dipropylene glycol | 1.0 |
| (4) | Amino-modified terpolymer emulsion (Synthetic Example 1) | 0.01 |
| (5) | Stearic acid monoglyceride | 1.0 |
| (6) | Glycerol | 2.5 |
| (7) | Liquid paraffin | 2.5 |
| (8) | Ethanol | 5.0 |
| (9) | Methylparaben | 0.1 |
| (10) | Perfume base | 0.1 |
| (11) | LPG (4.0 $kg/cm^2 \cdot G$, 20°C) | 10.0 |
| (12) | Water | Balance |
| | Total | 100 |

## Claims

1. A hair cosmetic composition comprising the following components (A) and (B):

   (A) an amino-modified terpolymer having structural units represented by the formulae (1), (2) and (3):

   $$[(R^1)_2SiO] \quad (1);$$

   $$[R^2R^3SiO] \quad (2);$$

   and

   $$[R^4SiO_{3/2}] \quad (3)$$

   wherein $R^1$, $R^2$ and $R^4$ are independently a monovalent hydrocarbon group having 1-20 carbon atoms, and $R^3$ is a group, $-R^5-(NR^6-R^7)_a-N^8R^9$ in which $R^5$ and $R^7$ are independently a divalent hydrocarbon group having 1-6 carbon atoms, $R^6$, $R^8$ and $R^9$ are independently a hydrogen atom or a monovalent hydrocarbon group having 1-20 carbon atoms, and a is a number of 0-3, wherein the molar ratio (1):(2):(3) of the structural units (1), (2) and (3) is 1:0.001-0.1:0.001-0.1, and at least one terminal of the terpolymer is capped with a hydroxyl group; and
   (B) one or more cationic non-silicone conditioning components.

2. The hair cosmetic composition according to Claim 1, wherein in the component (A), the molar ratio (1):(2):(3) of the structural units (1), (2) and (3) is 1:0.01-0.05:0.01-0.05.

3. The hair cosmetic composition according to Claim 1 or 2, wherein the component (A) is an emulsion obtained by emulsion polymerization.

4. The hair cosmetic composition according to any one of Claims 1 to 3, wherein the component (B) is at least one selected from the group consisting of cationic surfactants and water-soluble cationic polymers.

5. The hair cosmetic composition according to any one of Claims 1 to 4, wherein the component (A) and the component (B) are incorporated in a weight ratio of 1/100 to 100/1.

6. The hair cosmetic composition according to any one of Claims 1 to 5, further comprising at least one silicone derivative (C) other than the component (A).

7. The hair cosmetic composition according to Claim 6, wherein the silicone derivative (C) is at least one selected from the group consisting of dimethyl polysiloxanes, methylphenyl polysiloxanes, polyether-modified silicones, epoxy-modified silicones, fluorine-modified silicones, alcohol-modified silicones, alkyl-modified silicones, alkoxy-modified silicones and trimethylsiloxysilicic acids.

**Patentansprüche**

1. Haarkosmetische Zusammensetzung, umfassend die folgenden Komponenten (A) und (B):

(A) ein Amino-modifiziertes Terpolymer mit strukturellen Einheiten, dargestellt durch die Formeln (1), (2) und (3):

$$[(R^1)_2SiO) \quad (1);$$

$$[R^2R^3SiO] \quad (2);$$

und

$$[R^4SiO_{3/2}] \quad (3)$$

worin $R^1$, $R^2$ und $R^4$ unabhängig eine monovalente Kohlenwasserstoffgruppe mit 1 bis 20 Kohlenstoffatomen und $R^3$ eine Gruppe $-R^5-(NR^6-R^7)_a-NR^8R^9$ sind, worin $R^5$ und $R^7$ unabhängig eine bivalente Kohlenwasserstoffgruppe mit 1 - 6 Kohlenstoffatomen, $R^6$, $R^8$ und $R^9$ unabhängig ein Wasserstoffatom oder eine monovalente Kohlenwasserstoffgruppe mit 1 bis 20 Kohlenstoffatomen sind und a eine Zahl von 0 bis 3 ist, worin das molare Verhältnis (1):(2):(3) der strukturellen Einheiten (1), (2) und (3) 1:0,001-0,1:0,001-0,1 ist und zumindest ein Ende des Terpolymers mit einer Hydroxylgruppe versehen ist; und

(B) eine oder mehrere kationische Nicht-Silicon-Konditionierkomponenten.

2. Haarkosmetische Zusammensetzung nach Anspruch 1, worin in der Komponente (A) das molare Verhältnis (1):(2):(3) der strukturellen Einheiten (1), (2) und (3) 1:0,01-0,05:0,01-0,05 ist.

3. Haarkosmetische Zusammensetzung nach Anspruch 1 oder 2, worin die Komponente (A) eine Emulsion ist, die durch Emulsionspolymerisation erhalten ist.

4. Haarkosmetische Zusammensetzung nach einem der Ansprüche 1 bis 3, worin die Komponente (B) zumindest eine ist, ausgewählt aus der Gruppe, bestehend aus kationischen Tensiden und wasserlöslichen kationischen Polymeren.

5. Haarkosmetische Zusammensetzung nach einem der Ansprüche 1 bis 4, worin die Komponente (A) und die Komponente (B) in einem Gewichtsverhältnis von 1/100 bis 100/1 eingefügt sind.

6. Haarkosmetische Zusammensetzung nach einem der Ansprüche 1 bis 5, weiterhin umfassend zumindest ein anderes Siliconderivat (C) als die Komponente (A):

**7.** Haarkosmetische Zusammensetzung nach Anspruch 6, worin das Siliconderivat (C) zumindest eines ist, ausgewählt aus der Gruppe, bestehend aus Dimethylpolysiloxanen, Methylphenylpolysiloxanen, polyethermodifizierten Siliconen, epoxymodifizierten Siliconen, fluormodifizierten Siliconen, alkoholmodifizierten Siliconen, alkylmodifizierten Siliconen, alkoxymodifizierten Siliconen und Trimethylsiloxykieselsäuren.

**Revendications**

**1.** Composition cosmétique pour les cheveux comprenant les composants (A) et (B) suivants :

(A) un terpolymère modifié en amino comportant des motifs de structure représentés par les formules (1), (2) et (3) :

$$[(R^1)_2SiO] \quad (1),$$

$$[R^2R^3SiO] \quad (2),$$

et

$$[R^4SiO_{3/2}] \quad (3)$$

dans lesquelles $R^1$, $R^2$ et $R^4$ sont indépendamment un groupement hydrocarbure monovalent comportant 1 à 20 atomes de carbone, et $R^3$ est un groupement $-R^5-(NR^6-R^7)_a-NR^8R^9$ dans lequel $R^5$ et $R^7$ sont indépendamment un groupement hydrocarbure divalent comportant 1 à 6 atomes de carbone, $R^6$, $R^8$ et $R^9$ sont indépendamment un atome d'hydrogène ou un groupement hydrocarbure monovalent comportant 1 à 20 atomes de carbone, et a est un nombre de 0 à 3, dans lequel le rapport molaire (1):(2):(3) des motifs de structure (1), (2) et (3) est de 1:0,001 à 0,1:0,001 à 0,1, et au moins une terminaison du terpolymère est coiffée avec un groupement hydroxyle, et
(B) un ou plusieurs composants de conditionnement cationiques qui ne sont pas de la silicone.

**2.** Composition cosmétique pour les cheveux selon la revendication 1, dans laquelle dans le composant (A), le rapport molaire (1):(2):(3) des motifs de structure (1), (2) et (3) est de 1:0,01 à 0,05:0,01 à 0,05.

**3.** Composition cosmétique pour les cheveux selon la revendication 1 ou 2, dans laquelle le composant (A) est une émulsion obtenue par polymérisation en émulsion.

**4.** Composition cosmétique pour les cheveux selon l'une quelconque des revendications 1 à 3, dans laquelle le composant (B) est au moins un composé choisi parmi le groupe constitué de tensioactifs cationiques et de polymères cationiques solubles dans l'eau.

**5.** Composition cosmétique pour les cheveux selon l'une quelconque des revendications 1 à 4, dans laquelle le composant (A) et le composant (B) sont incorporés suivant un rapport massique de 1/100 à 100/1.

**6.** Composition cosmétique pour les cheveux selon l'une quelconque des revendications précédentes 1 à 5, comprenant en outre au moins un dérivé de silicone (C) autre que le composant (A).

**7.** Composition cosmétique pour les cheveux selon la revendication 6, dans laquelle le dérivé de silicone (C) est au moins un dérivé choisi parmi le groupe constitué de diméthylpolysiloxanes, de méthylphénylpolysiloxanes, de silicoses modifiées par un polyéther, de silicones modifiées par un époxy, de silicones modifiées par le fluor, de silicones modifiées par un alcool, de silicones modifiées par un alkyle, de silicones modifiées par un alcoxy et d'acides triméthylsiloxysiliciques.